# FASCICULE DE BREVET EUROPEEN

(11) **EP 4 090 938 B1**
(45) Date de publication et mention de la délivrance du brevet: **10.09.2025**
(21) Numéro de dépôt: 21705241.4
(22) Date de dépôt: 15.01.2021
(51) Int. Cl.: G01N 15/12, G06N 3/045, G01N 15/10

(54) **METHODE D'ANALYSES MEDICALES A TRAITEMENT DE SIGNAUX D'IMPEDANCE**
MEDIZINISCHES ANALYSEVERFAHREN MIT IMPEDANZSIGNALVERARBEITUNG
MEDICAL ANALYSIS METHOD WITH IMPEDANCE SIGNAL PROCESSING

(30) Priorité: 17.01.2020 FR 2000439
(43) Date de publication de la demande: 23.11.2022
(73) Titulaire: HORIBA ABX SAS, 34184 Montpellier (FR); UNIVERSITE DE MONTPELLIER, 34090 Montpellier (FR); Centre National de la Recherche Scientifique (CNRS), 75016 Paris (FR)
(72) Inventeur: TARACONAT, Pierre, 34184 MONTPELLIER (FR); ISEBE, Damien, 34184 MONTPELLIER (FR); MENDEZ, Simon, 75016 PARIS (FR); NICOUD, Franck, 34090 MONTPELLIER (FR)
(74) Mandataire: Cabinet Netter
(86) Numéro de dépôt international: PCT/FR2021/050078
(87) Numéro de publication internationale: WO 2021/144546

(56) Documents cités:
- EP-A1- 3 396 354
- EP-A1- 3 959 503
- WO-A1-2019/173233
- CN-A- 101 226 133
- US-A- 3 919 050
- US-A1- 2018 211 380
- NINGQUAN WANG ET AL: "Processing code-multiplexed Coulter signals via deep convolutional neural networks", LAB ON A CHIP, vol. 19, no. 19, 27 September 2019 (2019-09-27), pages 3292 - 3304, XP055749075, ISSN: 1473-0197, DOI: 10.1039/C9LC00597H
- THOMAS J S DURANT ET AL: "Very Deep Convolutional Neural Networks for Morphologic Classification of Erythrocytes", CLINICAL CHEMISTRY, vol. 63, no. 12, 1 December 2017 (2017-12-01), US, pages 1847 - 1855, XP055749079, ISSN: 0009-9147, DOI: 10.1373/clinchem.2017.276345

## Description

Depuis les années 1950, le comptage et la volumétrie des différentes cellules sanguines dans les automates d'hématologie sont effectués par mesure d'impédance, selon une méthode connue sous le nom de Principe Coulter. Cette méthode consiste à faire passer les cellules en suspension dans un liquide conducteur à travers un micro-orifice polarisé et à détecter les variations de résistance électrique (ou variations d'impédance) induites par les passages de particule dans l'orifice. La détection des différentes impulsions ainsi générées permet le comptage des éléments.

Différentes solutions ont été développées pour tenir compte des problèmes associés à la circulation dans un orifice (rotations due à des effets de bord ou hydrodynamiques, masquage de doublets, etc.). Comme aucune de ces solutions n'apportait satisfaction, la technologie de focalisation hydrodynamique, ou hydrofocalisation a été développée. Cette solution consiste à réaliser un gainage hydrodynamique du flux de cellules qu'on cherche à analyser, ce qui permet de le centrer dans l'orifice et limite les effets liés aux passages au bord. Cette technique est néanmoins très complexe à mettre en œuvre et particulièrement coûteuse.

La Demanderesse a récemment développé une solution permettant de fournir des résultats très satisfaisants tout en s'affranchissant de l'hydrofocalisation. Elle a protégé cette solution dans la demande de brevet FR 1904410. Au cours de ce développement, la Demanderesse s'est rendue compte que ses travaux pouvaient également être utilisés pour caractériser les cellules à partir de leurs signaux d'impédance, afin de retourner une information de normalité ou d'anormalité ou encore de caractériser la morphologie de ces cellules.

L'état de l'art sur les techniques existantes pour la mesure de la déformabilité des globules rouges révèle deux familles principales.

La première famille concerne des techniques de mesure précises mais longues et complexes à mettre en œuvre. Pour certaines d'entre elles, des modèles analytiques permettent de remonter à des paramètres rhéologiques tels que les modules élastiques et visqueux membranaire, entre autres. Parmi ces méthodes on peut citer l'aspiration par micropipette, dont le principe est d'aspirer une partie d'un globule rouge dans une pipette en imposant une dépression connue. En mesurant certaines quantités liées à la forme du globule rouge après aspiration, le module de cisaillement ou la viscosité de cisaillement peuvent être déduits (voir par exemple l'article de E. A. Evans « New membrane concept applied to the analysis of fluid shear and micropipette deformed red blood cells » Biophysical Journal, 1973). D'autres méthodes, comme la méthode *« optical tweezer »* (voir par exemple l'article de Brandao et al. « Optical tweezers for measuring red blood cell elasticity : application to the study of drug response in sickle cell disease » European Journal of Haematology, 70 :207-211, 2003), ou la méthode « *optical stretcher »* (voir par exemple l'article de Guck et al. « The optical stretcher : a novel laser tool to micromanipulate cells » Biophysical Journal, 81 :767-784, 2001) consistent à étirer le globule à l'aide de laser optiques et à observer sa forme.

La seconde famille inclut des techniques permettant de traiter de manière plus rapide et plus autonome un grand nombre de cellules et ainsi avoir une idée statistique de la déformabilité des globules rouges d'un échantillon. Ce type de technique étudie la déformation des globules rouges au moyen d'un paramètre de forme, nommé index de déformation (Deformation Index en anglais ou DI), qui est en fait une mesure de l'allongement du globule rouge soumis à une sollicitation mécanique bien connue. L'index de déformation combine les paramètres mécaniques et morphologiques du globule rouge : il est donc plus simple à étudier mais ne donne pas d'informations rhéologiques précises. Parmi ces techniques, on peut retenir celle présentée dans l'article de Cha et al. « Cell streching measurement utilizing viscoelastic particle focusing » Analytical chemistry 2012, dans lequel les globules rouges sont étirés à la chaîne dans un écoulement extensionnel et observés à l'aide d'une caméra. Sur le même principe, mais en sollicitant les globules rouges dans un écoulement de cisaillement, la méthode décrite dans l'article de Dobbe et al. « Analyzing red blood cell-deformability distributions », Blood Cells, Molecules, and Diseases, 28 :373-384, 2002, a permis de montrer l'impact de certaines pathologies sur les distributions de l'index de déformation des globules rouges. L'article de Mohandas et al. « Analysis of factors regulating erythrocyte deformability », Journal of Clinical Investigations, 66 :563-573, 1980, décrit un ektacytomètre dans lequel des globules rouges sont sollicités en écoulement cisaillé, et pour lesquels les spectres de diffraction de lumière sont observés pour mesurer l'index de déformation. En étudiant la courbe de l'index de déformation en fonction de l'osmolarité du milieu suspendant (voir par exemple l'article de Clark et al. « Osmotic gradient ektacytometry : comprehensive characterization of red cell volume and surface maintenance », Blood, 61 :899-910, 1983), il a été montré que certains paramètres rhéologiques et/ou morphologiques peuvent être mesurés.

Bien que plus riches en information, les méthodes de la première famille ne permettent pas de réaliser une analyse hématologique haute cadence. En effet, elles sont trop longues et complexes à mettre en œuvre et nécessitent l'intervention d'un manipulateur spécialisé. D'un autre côté, les méthodes de la deuxième famille, bien que plus aisées à mettre en œuvre, restent compliquées à industrialiser dans le cadre d'un laboratoire d'analyse médical, bien que l'étude d'une réponse plus globale au regard de l'indice de déformation qu'elles offrent permet d'isoler des sous populations de globules rouges pathologiques.

Un procédé d'analyse d'un échantillon sanguin est connu de US3919050A.

Toutes les méthodes citées ci-dessus nécessitent un système d'acquisition optique ou par vidéo-microscopie. Cela est nettement plus compliqué et couteux à mettre en œuvre qu'un système de mesure d'impédance. Néanmoins, les compteurs fonctionnant sous le Principe Coulter ne sont pas capables de déterminer l'indice de déformation ou une version simplifiée de celui-ci.

Il existe donc un besoin d'offrir un dispositif de mesure simple permettant de discriminer des populations de cellules selon leurs caractéristiques morphologiques.

L'invention vient améliorer la situation. A cet effet, elle concerne un procédé de classification d'un échantillon sanguin selon la revendication indépendante 1 comprenant les opérations suivantes :
a) recevoir des jeux de données de pulse, chaque jeu de données de pulse comprenant des données de valeur d'impédance associées à chaque fois à un marqueur de temps, ces données représentant ensemble une courbe de valeurs d'impédance cellulaire mesurées lors du passage d'une cellule dans un orifice polarisé, les cellules étant issues de l'échantillon sanguin,
b) pour chaque jeu de données de pulse
   b1. déterminer une valeur d'impédance maximale du jeu de données de pulse,
   b2. calculer une valeur d'impédance haute en multipliant la valeur d'impédance maximale par un coefficient haut choisi dans la plage [0,7 ; 0,95], et en déterminant dans le jeu de données de pulse les marqueurs de temps dont la valeur d'impédance associée dans le jeu de données de pulse est égale à la valeur d'impédance haute, et en calculant une durée haute correspondant à la durée maximale entre ces marqueurs de temps, et une valeur d'impédance basse en multipliant la valeur d'impédance maximale par un coefficient bas choisi dans la plage [0,1 ; 0,6], et en déterminant dans le jeu de données de pulse les marqueurs de temps dont la valeur d'impédance associée dans le jeu de données de pulse est égale à la valeur d'impédance basse, et en calculant une durée basse correspondant à la durée maximale entre ces marqueurs de temps,
   b3. calculer une valeur de position de pic égale à la division de la différence entre l'instant associée la valeur d'impédance maximale et le premier instant qui correspond à la valeur d'impédance basse, et la durée basse, et optionnellement une valeur de rotation égale la division de la durée haute par la durée basse,
c) déterminer la répartition statistique des jeux de données de pulses en fonction des couples durée basse/valeur de position de pic ou optionnellement des couples valeur de rotation/valeur de position de pic les statistiques étant établies par rapport à un jeu de plages de valeurs de couples,
d) classifier l'échantillon sanguin en comparant la répartition de l'opération c) à une répartition référence d'un échantillon de sang sain.

D'autres caractéristiques et avantages de l'invention apparaîtront mieux à la lecture de la description qui suit, tirée d'exemples donnés à titre illustratif et non limitatif, tirés des dessins sur lesquels :
- La figure 1 représente une vue de principe de l'orifice de mesure dans le cadre de l'invention, ainsi que des trajectoires que peut prendre une cellule dans celui-ci,
- La figure 2 représente des mesures de pulses pour les trajectoires de la figure 1,
- La figure 3 et la figure 4 représentent des diagrammes de caractérisation de pulses d'impédance de globules rouges mesurés avec l'agencement de la figure 1,
- La figure 5 représente un diagramme de caractérisation de zones d'intérêt,
- Les figures 6 à 9 représentent des proportions de pulses dans certaines des zones d'intérêt de la figure 5,
- La figure 10 représente un schéma d'un exemple de dispositif qui ne fait pas partie de l'invention,
- La figure 11 représente un schéma d'un réseau de neurone mis en œuvre dans l'exemple de la figure 10,
- La figure 12 représente un schéma d'un réseau de neurone mis en œuvre dans un autre exemple,
- La figure 13 représente un diagramme de caractérisation de zones d'intérêt,
- Les figures 14 à 16 représentent des représentations de statistiques de populations de pulses dans certaines des zones d'intérêt de la figure 13, et
- La figure 17 représente l'analyse de deux échantillons de sang sains sur une durée de 11 jours, et
- La figure 18 représente l'évolution statistique dans le temps des mesures de la figure 17 correspondant à la Box3' de la figure 5.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

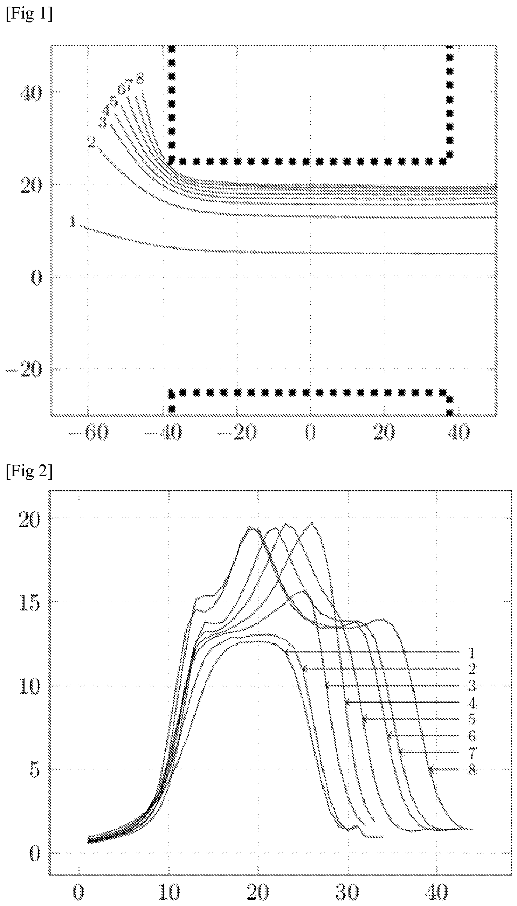

La figure 1 représente une vue de principe de l'orifice de mesure dans le cadre de l'invention, ainsi que des trajectoires que peut prendre une cellule dans celui-ci. L'orifice présente des parois représentées en pointillés, l'abscisse et l'ordonnée s'exprimant en µm.

La figure 2 représente les pulses d'impédance mesurés (tirés du signal correspondant à la variation d'impédance du système dû au passage d'une cellule dans le micro-orifice) pour chacune des trajectoires de la figure 1. L'abscisse s'exprime en µs, tandis que l'ordonnée s'exprime en ohm. Comme on peut le voir, plus la cellule a une trajectoire incidente près d'une des parois de l'orifice, plus la mesure est chaotique, et cause d'erreurs expliquées en introduction.

Les travaux de la Demanderesse exposés dans la demande FR 1904410 l'ont amenée à développer une nouvelle grandeur pour caractériser les pulses d'impédance. Cette grandeur est appelée WR, et c'est un ratio entre deux largeurs de pulse. Ces largeurs permettent d'indiquer la présence d'un pic dans le pulse, ou au contraire un pulse en forme de cloche.

Pour cela, on détermine d'abord la hauteur maximale du pulse dans le jeu de données de pulse. La hauteur maximale est utilisée pour calculer une valeur d'impédance haute et une valeur d'impédance basse.

La valeur d'impédance haute est obtenue en multipliant la valeur d'impédance maximale (qui correspond à la hauteur maximale) par un coefficient haut. Ce coefficient haut sert à déterminer deux instants qui, en général, permettent de bien approximer la largeur du pic d'impédance d'un pulse. Pour cela, le coefficient haut est choisi dans la plage [0,7 ; 0,95], et préférentiellement [0,8 ; 0,9], qui assure d'avoir au moins deux instants, et que ces instants correspondent bien au pic des pulses (afin de limiter les cas où plusieurs pics sont présents).

Ainsi la valeur d'impédance haute est inférieure à la valeur d'impédance maximale et supérieure à 70% de celle-ci. Les travaux de la Demanderesse ont montré que cette plage permet de bien capturer les pics des pulses produits. La Demanderesse a identifié que la valeur de 0,875 est particulièrement avantageuse et donne les meilleurs résultats : elle permet d'estimer de la manière la plus précise les pics des pulses. En effet, les pics autour de la hauteur maximale sont en général assez étroits.

La valeur d'impédance basse est obtenue en multipliant la valeur d'impédance maximale par un coefficient bas. Ce coefficient bas sert à déterminer deux instants qui, en général, permettent de bien approximer la largeur du pulse d'impédance. Pour cela, le coefficient bas est choisi dans la plage [0,1 ; 0,6], et préférentiellement [0,3 ; 0,6] qui assure d'avoir deux instants, et que ces instants correspondent à la largeur générale du pulse.

Ainsi la valeur d'impédance basse est comprise entre 30% et 60% de la valeur d'impédance maximale. Les travaux de la Demanderesse ont montré que cette plage permet de bien capturer la largeur des pulses produits en éliminant le bruit. La Demanderesse a identifié que la valeur de 0,5 est particulièrement avantageuse et donne les meilleurs résultats : les pentes des pulses en dessous de 50% de la hauteur maximale sont très abruptes, et cette valeur permet d'éviter tout risque de mesure bruitée.

Une fois la valeur d'impédance haute et la valeur d'impédance basse déterminées, on détermine la durée entre les deux instants du jeu de données de pulse qui sont les plus éloignés temporellement entre eux, et qui ont respectivement la valeur d'impédance haute ou la valeur d'impédance basse. La durée associée à la valeur d'impédance haute est appelée durée haute, et la durée associée à la valeur d'impédance basse est appelée durée basse. Instinctivement, il apparaît que la durée haute correspond sensiblement à la largeur du pic d'impédance d'un jeu de données de pulse, et que la durée basse correspond sensiblement à la largeur de pulse. Enfin, on détermine la grandeur WR en réalisant le rapport entre la durée haute et la durée basse.

La deuxième grandeur est appelée PP et est associée au pic du pulse. Pour cela, cette grandeur est calculée en faisant le ratio entre la différence entre l'instant où le pulse est maximal et le premier instant qui correspond à l'impédance basse, et la durée basse. Le résultat est un pourcentage qui indique la position du maximum de pulse dans celui-ci.

La Demanderesse a travaillé sur les représentations des pulses, notamment en établissant des graphes de type (PP ; WR). En effet, elle a découvert que ce type de graphe lui permettait d'identifier des comportements intéressants, en particulier pour les pulses caractéristiques de cellules ayant fait l'objet d'une rotation.

Afin de valider ses hypothèses, la Demanderesse a altéré la morphologie des globules rouges en ajoutant dans la solution électrolytique des molécules spécifiques.

Différentes concentrations de glutaraldéhyde et de N-Dodécyl-N,N-diméthyl-3-ammonio-1-propanesulfonate (également appelé sulfobétaïne 3-12, ci-après SB3-12), ont été ajoutées dans le réactif de dilution, puis les impédances de globules rouges d'un sang sain plongés dans ces solutions ont été mesurées. Plus précisément, les préparations comprenant du glutaraldéhyde à des concentrations comprises entre 0% et 0,5% ont été préparées d'une part, et des solutions comprenant du SB3-12 à des concentrations comprises entre 0 mg/L et 90 mg/L ont été préparées d'autre part. Les préparations ont été réalisées séparément, c'est-à-dire qu'elles ne contiennent chacune qu'un ajout de glutaraldéhyde ou qu'un ajout de SB3-12.

Il est connu que l'utilisation du glutaraldéhyde a un effet fixateur et permet de rigidifier les globules rouges tout en conservant leur forme discocyte. De même, il est connu que l'utilisation du SB3-12 tend à sphériser les cellules.

Un échantillon de sang provenant d'un patient sain (pour lequel il a été vérifié qu'il ne présentait aucune anomalie, appelé « sang sain » ci-après) a été analysé avec les différentes concentrations de SB3-12, et un autre a été analysé avec toutes les différentes concentrations de glutaraldéhyde. Chaque acquisition a été effectuée deux fois, pour une évaluation préliminaire de la répétabilité des développements proposés.

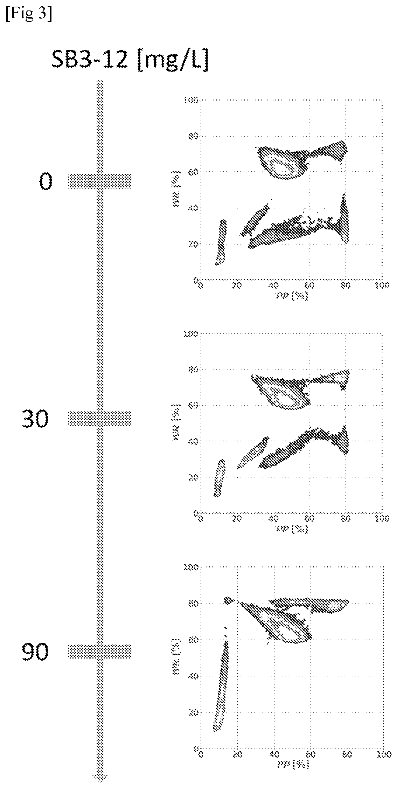

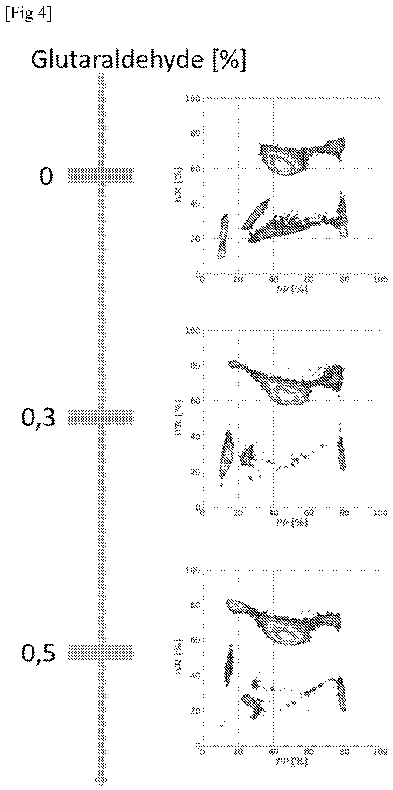

Enfin, la Demanderesse a calculé pour chaque préparation le graphe (PP ; WR) des pulses qui ont été générés. La figure 3 représente les graphes obtenus pour les préparations intégrant du SB3-12, tandis que la figure 4 représente les graphes obtenus pour les préparations intégrant du glutaraldéhyde.

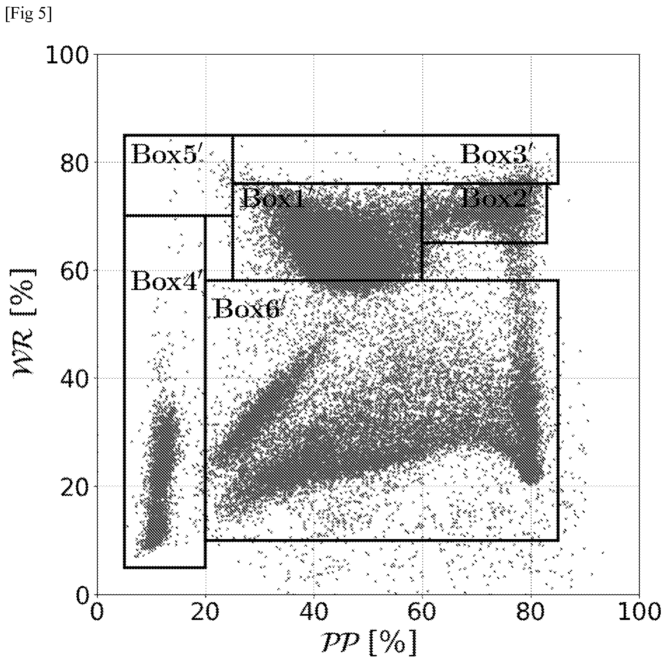

Ces graphes ont validé les intuitions de la Demanderesse, à savoir qu'ils contiennent des informations sur les caractéristiques morphologiques des globules rouges. Ainsi, la Demanderesse a établi des zones de pulses (Box1' à Box6') à étudier à partir du graphe (PP ; WR) d'un sang sain, comme représenté sur la figure 5. Les zones de pulses de la figure 5 ont été choisies afin de mettre en évidence les différences entre les acquisitions avec le glutaraldéhyde et les acquisitions avec le SB3-12 (voir figure 3 et figure 4).

Sur la figure 5, les zones de pulses ont été définies comme suit, chaque couple exprimant une plage PP (min ; max) et WR (min ; max) :
Box1' : (25 ; 60) - (58 ; 76)
Box2' : (60 ; 83) - (65 ; 76)
Box3' : (25 , 85) - (76 , 86)
Box4' : (5 , 20) - (5 , 70)
Box5' : (5 , 25) - (70 , 85)
Box6' : (20 , 85) - (10 , 58)

Dans chaque zone de la figure 5, la Demanderesse a calculé la proportion des pulses, et les moyennes des grandeurs PP et WR. Pour un échantillon donné, on a donc 18 paramètres au total (3 paramètres pour chacune des 6 zones).

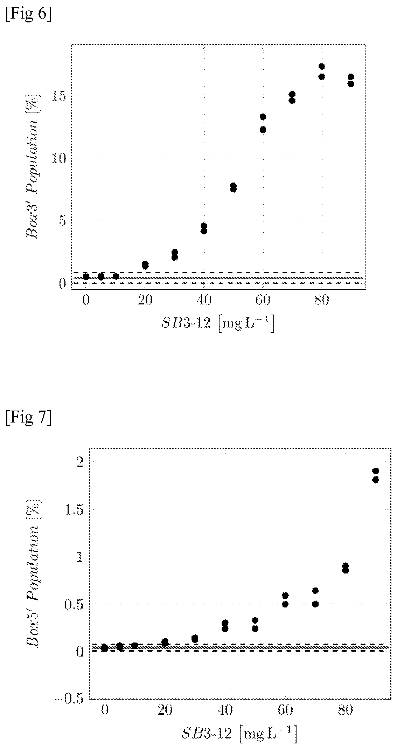

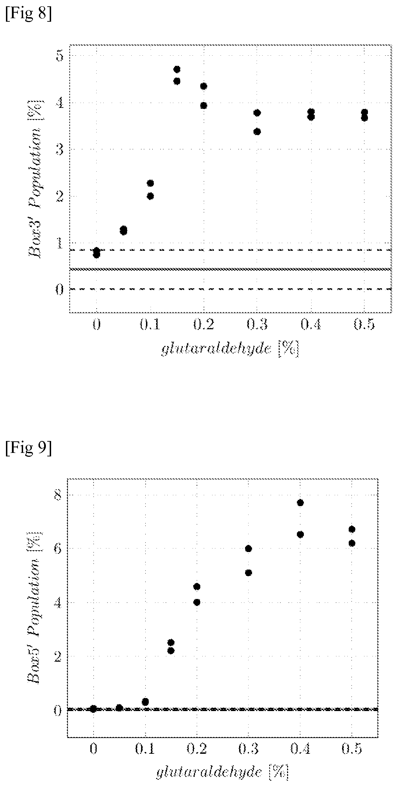

Ensuite, les évolutions de la proportion de pulses dans la zone Box3' et la zone Box5' ont été représentées, en fonction de la concentration en SB3-12 (sur les figures 6 et 7 respectivement), et en fonction de la concentration en glutaraldéhyde (sur les figures 8 et 9, respectivement).

Dans chacune des figures 6 à 9, la normalité est représentée par des lignes horizontales. La marge d'erreur est représentée par les lignes en pointillés, et est définie comme étant égale à deux fois l'écart type. Le trait continu entre les traits en pointillés représente la moyenne évaluée sur 22 sangs sains définissant la normalité.

L'analyse des figures 6 à 9 révèle que lorsque l'on augmente la concentration en SB3-12 ou en glutaraldéhyde, les paramètres calculés sortent de la normalité. Ces figures montrent clairement l'influence du SB3-12 et du glutaraldéhyde sur les globules rouges, et comment ils changent les caractéristiques morphologiques de ces derniers de manière visible dans les pulses.

En faisant l'hypothèse que la concentration en glutaraldéhyde et la concentration en SB3-12 sont respectivement corrélées à la rigidité et à la sphéricité des globules rouges, il semble possible de mesurer ces paramètres. En fait, en combinant les paramètres de proportion des pulses et de moyennes de la grandeur PP pour la zone 3' sur un graphe, il devient possible de distinguer de manière quantitative entre les globules rouges ayant été mélangés avec une préparation de glutaraldéhyde de ceux ayant été mélangés avec une préparation de SB3-12.

Tous ces éléments ont permis de valider de manière empirique le fait que les pulses d'impédances contiennent des informations concernant les caractéristiques morphologiques des globules rouges, mais il ne semble pas exister de fonction simple permettant de mesurer la normalité de globules rouges, ni de caractériser précisément leur anormalité morphologique le cas échéant.

La Demanderesse a donc eu l'idée de développer un dispositif utilisant un premier réseau de neurones entraîné et configuré pour indiquer la normalité ou l'anormalité d'une cellule sur la base de son pulse d'impédance, et un dispositif utilisant un deuxième réseau de neurones entraîné et configuré pour classifier une cellule en indiquant si elle présente des caractéristiques morphologiques normales, des caractéristiques morphologiques de cellule rigidifiée, ou des caractéristiques morphologiques de cellule sphérisée.

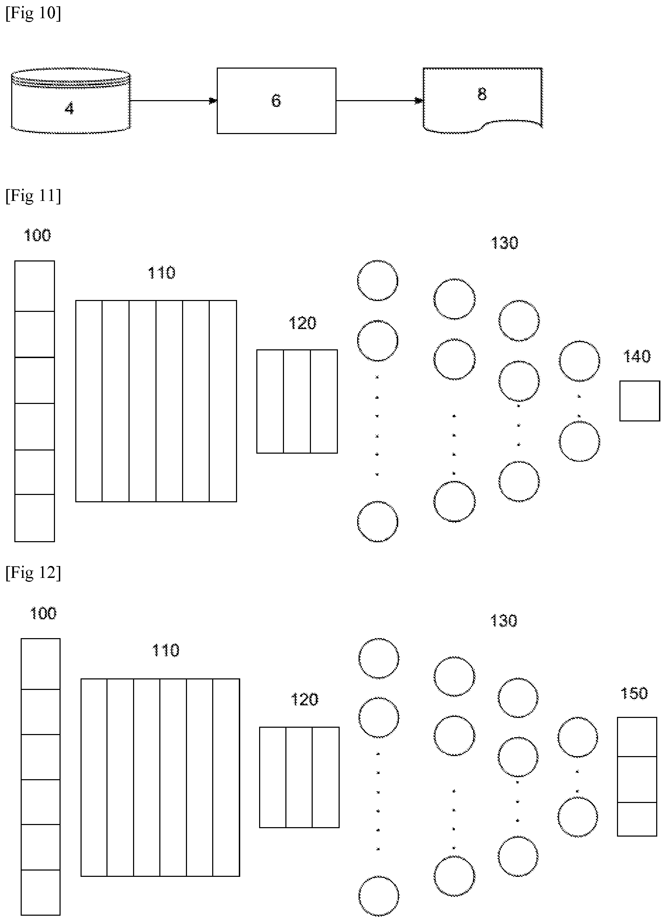

La figure 10 représente un schéma générique de ce dispositif. Le dispositif comprend une mémoire 4, et un classificateur (ou classifieur) 6.

La mémoire 4 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données calculées par le dispositif peuvent être stockées sur tout type de mémoire similaire à la mémoire 4, ou sur celle-ci. Ces données peuvent être effacées après que le dispositif a effectué ses tâches ou conservées.

Dans l'exemple décrit ici, la mémoire 4 reçoit des jeux de données de pulse. Un jeu de données de pulse représente l'ensemble des données qui permettent de caractériser un pulse d'impédance représenté sur la figure 2. C'est donc un ensemble de couples (valeur d'impédance mesurée ; marqueur de temps), qui définissent ensemble une courbe comme celles de la figure 2. Dans la pratique, le jeu de données de pulse sera en général un échantillonnage de la sortie de détection de l'orifice. Le jeu de données de pulse peut également être une courbe continue, auquel cas le calculateur 6 sera adapté en conséquence.

Le classificateur 6 est un élément accédant directement ou indirectement à la mémoire 4. Il peut être réalisé sous la forme d'un code informatique approprié exécuté sur un ou plusieurs processeurs. Par processeurs, il doit être compris tout processeur adapté aux calculs décrits plus bas. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille ou dans le cloud, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée.

Il convient de noter que le dispositif peut avantageusement être intégré dans un dispositif d'analyse hématologique, ou être déporté. Il peut donc être totalement intégré au dispositif d'analyse hématologique ou par exemple être un service Web auquel le dispositif d'analyse hématologique se connecte lorsque nécessaire ou souhaité.

Comme suggéré plus haut, le classificateur 6 est un réseau neuronal. En effet, les pulses peuvent être assimilés à des images, et, avec un entraînement adapté, la Demanderesse a considéré qu'un réseau neuronal pourrait être particulièrement performant pour classifier les pulses en ensemble de jeux de données de pulse avec une rotation ou sans rotation.

Plus particulièrement, la Demanderesse a identifié qu'un réseau de neurones à convolution était le mieux adapté. Ainsi, l'architecture du premier réseau de neurones est représentée sur la figure 11, tandis que celle du deuxième réseau de neurones est représentée sur la figure 12.

Dans les deux cas, le réseau de neurones est un réseau de neurones à convolution qui comprend deux couches de convolution. Ainsi, un jeu de données de pulse 100 (composé de 50 variables) est traité par une première couche de convolution 110 qui extrait 6 caractéristiques, puis une deuxième couche de convolution 120 extrait 3 caractéristiques à partir de la couche 110.

Les filtres (ou noyaux de convolution) de la première couche de convolution 110 ont une taille de 8, ceux de la deuxième couche de convolution 120 ont quant à eux une taille de 3.

La couche de convolution 120 est reliée à une couche entièrement connectée 130 du réseau neuronal qui comprend un enchaînement de 4 couches de neurones comprenant respectivement 80, 40, 20 et enfin 10 neurones.

Dans le cas du premier réseau de neurones, la couche entièrement connectée 130 retourne une valeur 140 dans la couche de sortie. Dans l'exemple décrit ici, la valeur 140 vaut 1 si la cellule est normale et 0 si elle est anormale.

Pour l'ensemble des neurones composant les différentes couches du modèle, la fonction d'activation retenue est la fonction sigmoïde.

Pour ce premier réseau de neurones, l'entraînement a été réalisé à l'aide de données provenant des acquisitions de sang sain définissant la normalité, d'acquisitions avec des concentrations en SB3-12 comprises entre 50 mg/L et 90 mg/L, et des acquisitions avec des concentrations en glutaraldéhyde comprises entre 0,3% et 0,5%. Le réseau de neurones a donc été entrainé à détecter des cellules très impactées. A chaque fois, les pulses d'entraînement avaient été labellisés avec la valeur 1 si la cellule associée était normale et avec la valeur 0 si la cellule associée était anormale.

La vérification de la pertinence de l'entraînement a été réalisée en écartant de l'entraînement certaines données et en les introduisant dans le réseau de neurones entraîné. Les résultats ont été excellents, et, avec un seuil à 0,5 sur la couche de sortie (c'est-à-dire que la valeur 1 est retournée si la couche de sortie retourne une valeur supérieure à 0,5 et 0 sinon), le taux de faux positifs était de 4,3% et le taux de faux négatifs de 3,1% sur le jeu de pulses de validation.

Dans le cas du deuxième réseau de neurones, la couche entièrement connectée 130 retourne un triplet 150 dans la couche de sortie. Dans l'exemple décrit ici, les trois composantes du triplet prennent pour valeur 0 ou 1.

Pour ce deuxième réseau de neurones, l'entraînement est réalisé de manière similaire au premier réseau de neurones, à l'exception près que les pulses d'entraînement sont labellisés avec des triplets indiquant si un pulse donné est normal ([1 ; 0 ; 0]), avec des caractéristiques morphologiques de sphérisation ([0 ; 1 ; 0]) ou avec des caractéristiques morphologiques de rigidification ([0 ; 0 ; 1]).

La vérification de la pertinence de l'entraînement a été réalisée en écartant de l'entraînement certaines données et en les introduisant dans le réseau de neurones entraîné. Les résultats ont été excellents, chaque élément sur la couche de sortie étant réduit à sa composante maximale, c'est-à-dire que [0,92 ; 0,02 ; 0,06] retourne le triplet [1 ; 0 ; 0], [0,01 ; 0,99 ; 0] retourne le triplet [0 ;1 ; 0], et [0,05 ; 0,25 ; 0,7] retourne le triplet [0 ; 0 ; 1]. Dans ces conditions, sur le jeu de pulses de validation, le taux de faux positifs était de 4% sur les cellules classifiées normales, de 7,5% sur les cellules classifiées à caractéristiques morphologiques de sphérisation, et de 8,2% sur les cellules classifiées à caractéristiques morphologiques de rigidification.

Ces résultats sont excellents et démontrent la pertinence du dispositif selon l'invention, qui permet d'obtenir des résultats d'une extrême précision avec des simples mesures d'impédance sans hydrofocalisation.

Les travaux de la Demanderesse lui ont permis d'établir qu'une unique couche de convolution pourrait suffire, ainsi qu'une couche entièrement connectée qui ne contiendrait que 2 couches ou moins.

En variante, la Demanderesse considère possible d'utiliser un perceptron multicouche (MLP) à la place du réseau de neurones convolutif décrit plus haut. En effet, même si ce type de réseau de neurones fournit des résultats moins précis à nombre de paramètres équivalent (typiquement, 5 à 8% de faux positifs supplémentaires), il constitue néanmoins une alternative plausible.

Ces résultats ont ouvert le chemin à la détection de pathologies qui ont pour conséquence une modification des caractéristiques morphologiques des globules rouges ou d'autres cellules, comme la malaria ou encore la dépranocytose. A chaque fois, il suffit de tester des sangs sains et des sangs malades pour labelliser les pulses correspondant, et d'entraîner le réseau de neurones des figures 11 ou 12 avec ces données.

Par exemple, en analysant un échantillon de culture dans lequel tous les globules rouges sont parasités par la malaria, une série de signatures labellisées à cette pathologie est obtenue et permet de générer un classifieur spécifique à cette infection.

La Demanderesse a également identifié que le couplage de la durée basse avec la grandeur PP ou de la grandeur WR avec la grandeur PP permet de réaliser une distinction simple entre cellules normales et cellules anormales sur des bases statistiques. Cela permet par exemple de mettre en œuvre l'invention sans utiliser un réseau de neurones décrit plus bas.

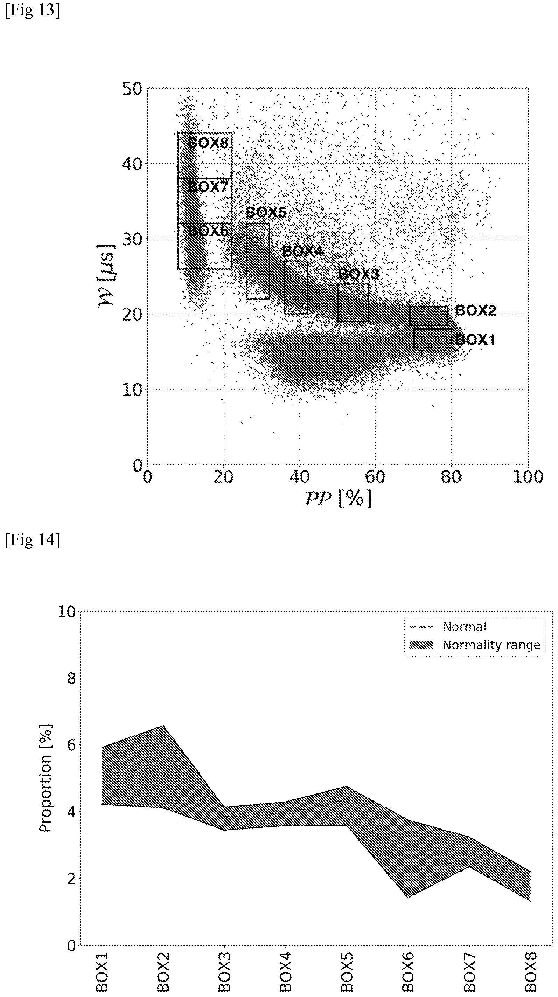

Ainsi, sur la base d'un ensemble de mesures sur du sang sain, la Demanderesse a établi la figure 13. Sur cette figure, la Demanderesse a regroupé toutes les grandeurs tirées des pulses, ce qui lui a permis de définir les zones de pulses Box1 à Box8.

Sur la figure 13, les zones de pulses ont été définies comme suit, chaque couple exprimant une plage PP (min ; max) et WR (min ; max) :
Box1 : (70 ; 80) - (15,5 ; 18)
Box2 : (69 ; 79) - (18,5 ; 21)
Box3 : (50 , 58) - (19 , 24)
Box4 : (36 , 42) - (20 , 27)
Box5 : (26 , 32) - (22 , 32)
Box6 : (8 , 22) - (26 , 32)
Box7 : (8 ; 22) - (32 ;38)
Box8 : (8 ; 22) - (38 ;44)

Ensuite, la Demanderesse a répété la même opération avec les jeux de données de pulses représentés sur la figure 3 et la figure 4 et a établi deux critères de normalité en comparant les statistiques de population pour chaque zone de pulses, pour le sang sain d'une part et pour les sangs des figures 3 et 4 d'autre part. En effet, la comparaison des statistiques de population permet d'établir des différences significatives pour chaque zone de pulses.

Ainsi, sur la figure 14, la Demanderesse a représenté d'une part les répartitions statistiques des populations pour chaque zone de pulse pour un sang sain (en traits pleins, définissant une zone en couleur pleine), et d'autre part la valeur d'un sang sain en traits tiretés.

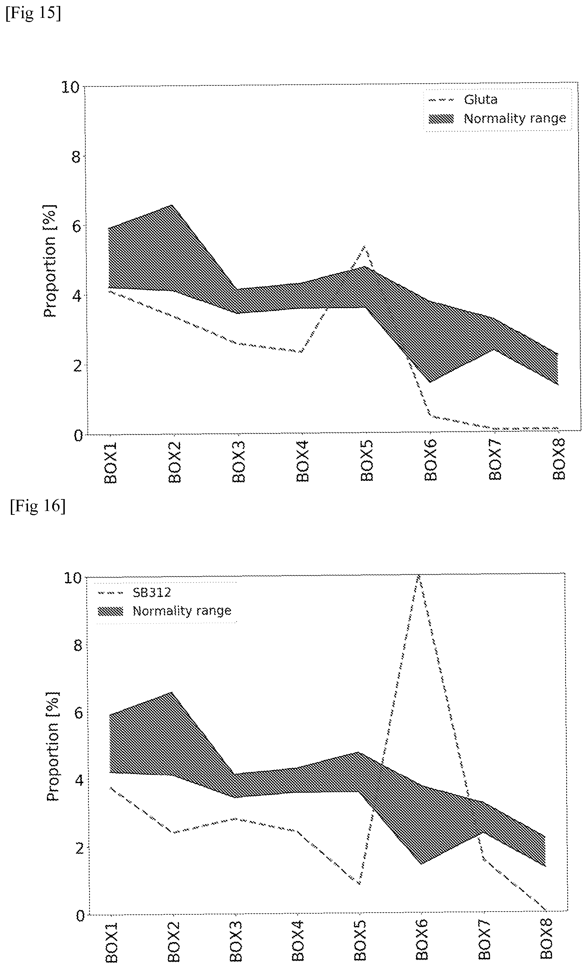

Sur la figure 15 (respectivement la figure 16), la Demanderesse a reproduit d'une part les répartitions statistiques des populations pour chaque zone de pulse pour un sang sain (en traits pleins, définissant une zone en couleur pleine, identiques à ceux de la figure 14), et d'autre part en traits tiretés ces mêmes répartitions mais pour des pulses obtenus sur un sang comprenant du glutaraldéhyde (respectivement du SB3-12).

Les figures 14 à 16 font clairement apparaître qu'il suffit de faire les statistiques de population pour les pulses tirés d'un échantillon de sang et de les comparer avec l'enveloppe de normalité définie par la figure 14 (et reproduite sur les figures 15 et 16) pour déterminer si l'échantillon est sain ou présente des propensions à la sphérisation ou la rigidification.

Ainsi, en analysant une portion d'un échantillon suffisante (par exemple environ 10 000 cellules, le volume du sang analysé étant dépendant des conditions de comptage), il est possible de retourner rapidement une information de type « échantillon de sang sain » ou « échantillon de sang anormal », sans utiliser de réseau neuronal.

En variante, au lieu du graphe (durée basse ; PP), on pourrait utiliser le graphe (WR ; PP) pour établir les zones de pulses permettant de définir les critères de normalité.

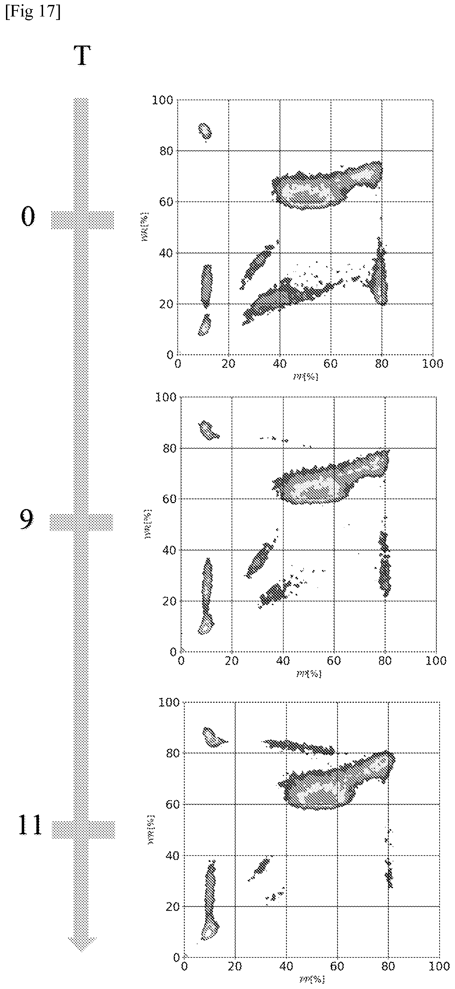

La figure 17 représente l'analyse de deux échantillons de sang sains sur une durée de 11 jours (passage en duplicate pour chaque analyse). Cette figure montre que la répartition des jeux de données de pulses sur un graphe WR/PP évolue en fonction de l'âge de l'échantillon.

Pour chaque analyse, la répartition statistique des jeux de données de pulses en fonction des couples WPP et WR/PP est calculée, les statistiques étant établies par rapport à un jeu de plage des métriques présenté sur les Fig. 5 et 13.

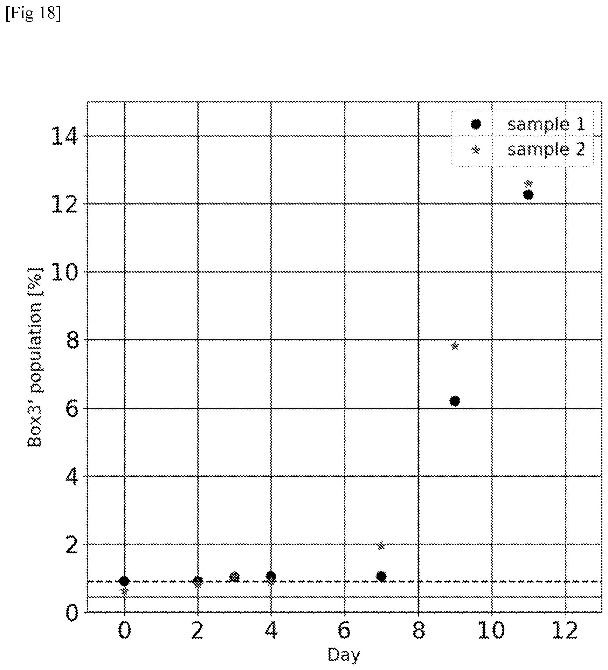

Sur la figure 18, seuls les résultats relatifs à BOX3' sont présentés par souci de concision. Cette figure représente l'évolution statistique dans le temps des pulses associés à la BOX3' de la Fig.5, en fonction de l'âge de l'échantillon, l'origine temporelle étant le jour du prélèvement de l'échantillon sur le patient.

Cette figure montre que les échantillons de sang restent « normaux » (n'évoluent pas) pendant 7 jours, puis deviennent « anormaux », dans le sens où le marqueur statistique se met à dériver fortement.

L'invention peut donc être utilisée pour réaliser un suivi de l'évolution dans le temps d'un échantillon de sang. Cette figure est conforme aux publications scientifiques du domaine : lorsque l'échantillon vieillit, les globules rouges présents dans l'échantillon voient leurs caractéristiques biomécaniques se dégrader. En particulier, il est connu que les globules rouges présentent une diminution de l'élasticité avec le temps. L'évolution dans le temps d'un échantillon peut être affecté par la durée de conservation, les conditions de conservation, etc. Dans tous les cas, les figures 17 et 18 montrent que le disposition selon l'invention permet de tirer une information relative à l'évolution dans le temps de l'échantillon, indicative par exemple de la validité de cet échantillon.

Bien que ce qui précède fasse principalement référence à l'étude de globules rouges, l'invention trouverait à s'appliquer à tout autre type de cellules dont les caractéristiques morphologiques sont susceptibles de changer, comme les plaquettes par exemple.

## Revendications

1. Procédé de classification d'un échantillon sanguin comprenant les opérations suivantes :
a) recevoir des jeux de données de pulse, chaque jeu de données de pulse comprenant des données de valeur d'impédance associées à chaque fois à un marqueur de temps, ces données représentant ensemble une courbe de valeurs d'impédance cellulaire mesurées lors du passage d'une cellule dans un orifice polarisé, les cellules étant issues de l'échantillon sanguin,
b) pour chaque jeu de données de pulse
b1. déterminer une valeur d'impédance maximale du jeu de données de pulse, le procédé étant **caractérisé en ce qu'**il comprend les opérations supplémentaires suivantes:
b2. calculer une valeur d'impédance haute en multipliant la valeur d'impédance maximale par un coefficient haut choisi dans la plage [0,7 ; 0,95], et en déterminant dans le jeu de données de pulse les marqueurs de temps dont la valeur d'impédance associée dans le jeu de données de pulse est égale à la valeur d'impédance haute, et en calculant une durée haute correspondant à la durée maximale entre ces marqueurs de temps, et une valeur d'impédance basse en multipliant la valeur d'impédance maximale par un coefficient bas choisi dans la plage [0,1 ; 0,6], et en déterminant dans le jeu de données de pulse les marqueurs de temps dont la valeur d'impédance associée dans le jeu de données de pulse est égale à la valeur d'impédance basse, et en calculant une durée basse correspondant à la durée maximale entre ces marqueurs de temps,
b3. calculer une valeur de position de pic (PP) égale à la division de la différence entre l'instant associée la valeur d'impédance maximale et le premier instant qui correspond à la valeur d'impédance basse, et la durée basse, et optionnellement une valeur de rotation (WR) égale la division de la durée haute par la durée basse,
c) déterminer la répartition statistique des jeux de données de pulses en fonction des couples durée basse/valeur de position de pic (PP) ou optionnellement des couples valeur de rotation/valeur de position de pic (WR) les statistiques étant établies par rapport à un jeu de plages de valeurs de couples (Box 1, Box 2, Box 3, Box 4, Box 5, Box 6, Box 7, Box 8, Box 1', Box 2', Box 3', Box 4', Box 5', Box 6'),
d) classifier l'échantillon sanguin en comparant la répartition de l'opération c) à une répartition référence d'un échantillon de sang sain.

## Patentansprüche

1. Verfahren zum Klassifizieren einer Blutprobe, das die folgenden Schritte umfasst:
a) Empfangen von Pulsdatensätzen, wobei jeder Pulsdatensatz Impedanzwertdaten umfasst, die jeweils einem Zeitmarker zugeordnet sind, wobei diese Daten zusammen eine Kurve von Zellimpedanzwerten darstellen, die beim Passieren einer Zelle durch ein polarisiertes Loch gemessen werden, wobei die Zellen aus der Blutprobe stammen,
b) für jeden Pulsdatensatz
b1. Bestimmen eines maximalen Impedanzwerts des Pulsdatensatzes, wobei das Verfahren **dadurch gekennzeichnet ist, dass** es die folgenden zusätzlichen Schritte umfasst:
b2. Berechnen eines hohen Impedanzwerts, indem der maximale Impedanzwert mit einem hohen Koeffizienten, der im Bereich [0,7; 0,95] ausgewählt ist, multipliziert wird und im Pulsdatensatz die Zeitmarker, deren zugehöriger Impedanzwert im Pulsdatensatz gleich dem hohen Impedanzwert ist, bestimmt werden, und eine hohe Dauer, die der maximalen Dauer zwischen diesen Zeitmarkern entspricht, berechnet wird, und eines niedrigen Impedanzwerts, indem der maximale Impedanzwert mit einem niedrigen Koeffizienten, der im Bereich [0,1; 0,6] ausgewählt ist, multipliziert wird und im Pulsdatensatz die Zeitmarker, deren zugehöriger Impedanzwert im Pulsdatensatz gleich dem niedrigen Impedanzwert ist, bestimmt werden und eine niedrige Dauer, die der maximalen Dauer zwischen diesen Zeitmarkern entspricht, berechnet wird,
b3. Berechnen eines Peak-Positionswerts (PP), der gleich der Division der Differenz zwischen dem Zeitpunkt, der dem maximalen Impedanzwert zugeordnet ist, und dem ersten Zeitpunkt, der dem niedrigen Impedanzwert entspricht, und der niedrigen Dauer ist, und optional eines Rotationswerts (WR), der gleich der Division der hohen Dauer durch die niedrige Dauer ist,
c) Bestimmen der statistischen Verteilung der Pulsdatensätze in Abhängigkeit von den Paaren niedrige Dauer/Peak-Positionswert (PP) oder optional von den Paaren Drehwert/Peak-Positionswert (WR), wobei die Statistiken in Bezug auf einen Satz von Bereichen von Werten der Paare (Box 1, Box 2, Box 3, Box 4, Box 5, Box 6, Box 7, Box 8, Box 1', Box 2', Box 3', Box 4', Box 5', Box 6') aufgestellt werden,
d) Klassifizieren der Blutprobe, indem die Verteilung von Schritt c) mit einer Referenzverteilung einer gesunden Blutprobe verglichen wird.

## Claims

1. A method for classifying a blood sample comprising the following operations:
a) receiving pulse data sets, each pulse data set comprising impedance value data each time associated with a time marker, these data together representing a curve of cell impedance values measured when a cell passes through a polarised opening, the cells originating from the blood sample,
b) for each pulse data set
b1. determining a maximum impedance value of the pulse data set, the method being **characterised in that** it comprises the following additional operations:
b2. calculating a high impedance value by multiplying the maximum impedance value by a high coefficient chosen in the range [0.7; 0.95], and by determining in the pulse data set the time markers whose associated impedance value in the pulse data set is equal to the high impedance value, and by calculating a high duration corresponding to the maximum duration between these time markers, and a low impedance value by multiplying the maximum impedance value by a low coefficient chosen in the range [0.1; 0.6], and by determining in the pulse data set the time markers whose associated impedance value in the pulse data set is equal to the low impedance value, and by calculating a low duration corresponding to the maximum duration between these time markers,
b3. calculating a peak position value (PP) equal to the division of the difference between the instant associated with the maximum impedance value and the first instant that corresponds to the low impedance value, and the low duration, and optionally a rotation value (WR) equal to the division of the high duration by the low duration,
c) determining the statistical distribution of the pulse data sets according to the low duration/peak position value (PP) pairs or optionally the rotation value/peak position value (WR) pairs, the statistics being established with respect to a set of pair value ranges (Box 1, Box 2, Box 3, Box 4, Box 5, Box 6, Box 7, Box 8, Box 1', Box 2', Box 3', Box 4', Box 5', Box 6'),
d) classifying the blood sample by comparing the distribution of operation c) to a reference distribution of a sample of healthy blood.
